# EUROPEAN PATENT APPLICATION

(11) **EP 1 857 146 A1**
(43) Date of publication of application: **21.11.2007**
(21) Application number: 06010106.0
(22) Date of filing: 16.05.2006
(51) Int. Cl.: A61P 11/00, A61K 38/17

(54) **Treatment of respiratory disorders**

(71) Applicant: Fraunhofer-Gesellschaft zur Förderung der angewandten Forschung e.V., 80686 München (DE)
(72) Inventor: Braun, Armin, 30916 Isernhagen (DE)
(74) Representative: Vossius & Partner

(57) **Abstract**

There is provided the use of a polypeptide in the preparation of a medicament for the prevention or treatment of a respiratory disorder in a subject. The respiratory disorder may particularly be asthma, cough or inflammatory rhinitis.

## Description

### Field of invention

The invention relates to the use of a polypeptide in the preparation of a medicament for the prevention or treatment of a respiratory disorder in a subject. The invention particularly relates to the prevention or treatment of asthma, cough or inflammatory rhinitis

### Background

Chronic inflammatory lung diseases are a group of severe disorders, with asthma being the major example of such a disease. Asthma is a common condition in the Western world, with over 3 million people in the United Kingdom and approximately 10 million people in the USA having the disease. Around 1500 people per year are thought to die in the UK as a result of the disease. The airways of an asthma sufferer are permanently inflamed; when the sufferer then comes into contact with an allergen or other airways irritant, the airways become further inflamed and hence narrowed, making it harder for the sufferer to breathe. Symptoms of asthma include coughing, wheezing, tight chestedness and breathlessness.

Asthma cannot be cured but can be treated in two ways, by separate "reliever" and "preventer" treatments. Both treatments are usually delivered via an inhaler device which administers either a liquid aerosol or a fine powder, for inhalation by the user. "Reliever" treatments are used when asthma symptoms occur and comprise a bronchodilator medicine such as salbutamol and terbutaline. These medicines relieve the symptoms of an asthma attack quickly. "Preventer" treatments are used regularly, even in the absence of symptoms, to prevent an asthma attack from occurring.

Preventers usually contain a steroid which works, long term, to reduce airway inflammation. Preventer treatment may also be administered by oral tablets.

Side effects of these medicines are mild and rare. High doses of reliever treatment canresult in an increased heart rate and physical shakes. Use of a preventer inhaler can result in oral thrush infection and hoarseness of the voice, though this can usuallybeavoided by rinsing the mouth after inhaler use. There have been reports that the regular use of preventer inhalers is associated with increased risk of death from asthma, whilst use of reliever inhalers is associated with decreased risk (Lanes et al. (2002) Thorax 57 683-686; Suissa et al. (2000) N. Engl. J. Med. 343 332-336). This is a reflection of the fact that preventer treatments seek to reduce the underlying causes of asthma attacks, whereas the reliever treatments merely alleviate the symptoms when they appear.

Rhinitis is an inflammation of the mucous membranes of the nose. Symptoms include itching, sneezing, excessive mucous production and congestion. Rhinitis may be non-allergic or allergic, the latter being triggered by various allergens such as pollen and dust mites. Seasonal allergic rhinitis is commonly referred to as "hay-fever. Non-allergic rhinitis may also be aggravated by irritants such as fumes, smoke and temperature changes. Decongestants may be used to alleviate the symptoms of rhinitis, as can the topical application of steroid compounds, although no medication can give complete relief from the symptoms of this condition.

### Pathogenesis of asthma

Allergic bronchial asthma is characterised by chronic airway inflammation, recurrent reversible broncho-obstruction and development of airway hyperresponsiveness (AHR) in response to a wide range of stimuli, including methacholine, histamine, cold air and cigarette smoke. When a susceptible individual is repeatedly exposed to such allergens, that individual develops specific IgE antibodies to the allergens. Subsequent re-exposure to the same agent causes a two-stage reversible airway obstruction referred to as the allergic early and late phase responses.

The early phase response (EAR) begins within minutes from the start of exposure to the allergen and is characterised by a rapid onset of mucosal swelling. Airway smooth muscle tone is increased and mast cells become activated and degranulated (Pauwels (1989) Clin. Exp. Allergy 19 395-398; Turner % Kinet (1999) Nature 402 B24-B30). The mast cells secrete various factors including histamine, growth factors and cytokines and these factors are thought to act as the trigger for the onset of the allergic late phase reaction (Wasserman (1984) Environ. Health Perspect. 55 259-269).

The late phase response (LAR) takes place some hours after exposure to the allergen. It may continue for several days, even in the absence of re-exposure to the allergen. The airway is narrowed and eosinophils, neutrophils and lymphocytes move from the blood into the lung parenchyma and airway epithelium (de Monchy et al. (1985) Am. Rev. Respir. Dis. 131 373-376; Cockroft (1988) Chest 94 178-180; Pauwels (1989) Clin. Exp. Allergy 19 395-398; Bousquet et al. (1990) N. Engl. J. Med. 323 1033-1039). Once they have been activated and recruited to the airways in this way, the cells can induce a chronic inflammatory response which may be followed by structural changes to the lung (i.e. airway remodelling).

Human lungs are innervated via a complex network of autonomic neurons. Neuronal control of airway function depends on a balance between sensory input and motor output activities of these neurons. This balance appears to be under the close control of inflammatory mediators such as neurotrophins, cytokines, lipid mediators and mast-cell products. There is increasing evidence that dysfunction and dysregulation of this nervous system contributes to the pathogenesis of asthma and other chronic inflammatory lung diseases (reviewed in Braun et al (2002) Curr. Op. Pharmacol. 2 229-234).

### Neurotrophins and their role in the development of asthma

The neurotrophin family of peptides includes Nerve Growth Factor (NGF), Brain Derived Neurotrophic Factor (BDNF) and Neurotrophin-3 and -4 (NT-3 and NT-4). These factors are produced in nerve-associated cells, neurons and by a wide range of immune cells (Lewin & Barde (1996) Annu. Rev. Neurosci. 19 289-317; Levi-Montalcini et al. (1995) J. Neurol. Sci. 130 119-127; Leon et al. (1994) Proc. Natl. Acad Sci. USA. 91 3739-3743; Braun et al. (1998) Eur. J. Immunol. 28 3240-3251; Ehrhard et al. (1993) Proc. Natl. Acad. Sci. U.S.A. 90 10984-10988; Torcia et al. (1996) Cell 85 345-356). They are also produced by lung fibroblasts and epithelial cells (Kassel et al. (2001) Clin. Exp. Allergy 31 1432-1440: Olgart & Frossard (2001) Eur. Respir. J. 18 115-121; Fox et al. (2001) Eur. J. Pharmacol. 424 159-162).

NGF has been found to be elevated in the serum of patients with allergic diseases, the highest levels being found in patients suffering from asthma (Bonini et al. (1996) Proc. Natl. Acad. Sci. U.S.A. 93 10955-10960). Such elevated NGF levels are also found in bronchoalveolar lavage fluids (BALF) after segmental allergen provocation (Virchow et al. (1998) Am. J. Respir, Crit. Care Med. 158 2002-2005). These elevated levels are also seen in the nasal lavage fluids of patients with allergic rhinitis, the elevation increasing further in response to allergens (Sancio et al. (2000) Am. J. Crit. Care Med. 161 1631-1635).

It has been shown that transgenic mice engineered to express NGF from the lungspecific Clara-cell secretory protein (CCSP) promoter exhibit hyperinnevation of the airways. During acute lung inflammation induced by ozone inhalation, such animals display a two-fold increase in influx of neutophils into the airway (Graham et al. (2001) Am. J. Respir. Crit. Care Med. 164 307-313). These mice have also been shown to exhibit augmented airway inflammation compared to wild-type mice (Path et al. (2002) Am. J. Respir. Crit. Care Med. 166 818-826) and show increased AHR following exposure to allergens (Braun et al. (2001) Int. Arch Allergy Immunol. 124 205-207). In the same model mice carrying a mutation in the low-affinity NGF receptor (p75^{NGF}) showed a decrease of such neutrophilic inflammation of nearly 50%. p75^{NGF} knockout mice showed a deficit in airway innervation (Lec et al. (1992) Cell 69 737-749). In the context of asthma, such knockout mice show diminished allergen-induced allergic inflammation, associated with a lack of AHR (Tokuoka et al. (2001) Br. J. Pharmacol. 134 1580-1586).

In addition to the above results, it has been shown that, in a murine model of allergic airway inflammation, application of NGF into the airways induced the development of AHR (Braun et al. (2001) Int. Arch. Allergy Immunol. 124 205-207). Treatment with intravenous NGF increases histamine-induced acute bronchoconstriction in guinea-pigs (de Vries et al. (1999) Am. J. Respir. Crit. Care Med. 159 1541-1544).

Pre-treatment with anti-NGF diminished the EAR in both allergen-sensitized guinea pigs and in mice (Glaab et al. (2002) J. Appl. Physiol. 93 1208-1214; Päth et al. (2002) Am. J. Respir. Crit. Care Med 166 818-826). Glaab et al. further demonstrated that anti-NGF is sufficient to attenuate the magnitude of acute bronchoconstriction in a rat model of allergic asthma, without effecting the late asthmatic response or airway inflammation (Glaab et al. (2003) Allergy 58 900-904).

WO-A-02/096458 describes the use of anti-NGF antibodies for the treatment of various disorders. including asthma, arthritis and psoriasis. There is no mention of the use of polypeptides other than anti-NGF antibodies for such treatment.

WO-A-03/060471 relates to the diagnosis and treatment of IgE implicated disorders, including asthma. The application relates to the peptide LT-10, which has the amino acid sequence LKAMDPTPPL and which reduces IgE levels in humans with the apparent effect of bringing other proteins, including NGF, into homeostasis.

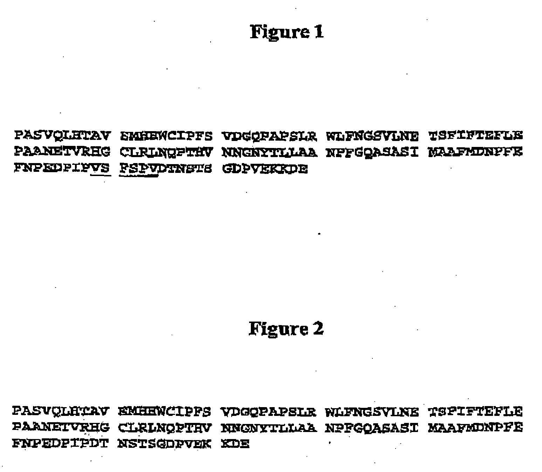

The polypeptide sequences of Figure I (polypeptide A) and Figure 2 (polypeptide B) are closely related polypeptides, with a six amino acid difference between their sequences (underlined in Figure 1). These polypeptides are functional fragments of the high-affinity receptor for NGF, tyrosine kinase A (TrkA). In WO-A-99/53055, it was shown that polypeptide B could bind to NGF in a competitive binding assay against p75^{NTR}. The polypeptide was shown to inhibit NGF-induced plasma extravasation in vivo, both when co-injected with NGF and when added as a pre-treatment 5 minutes or 40 minutes prior to NGF injection WO-A-03/025016 showed that a histidine-tagged version of this polypeptide can bind to NGF, inhibit pain responses in the guinea-pig and can prevent neurite outgrowth from PC12 cells in response to NGF; non-tagged polypeptide B was also shown to prevent such neurite outgrowth. In addition, that application showed that a histidine-tagged version of polypeptide A can bind to NGF with high affinity.

### SUMMARY OF THE INVENTION

According to a first aspect of the invention, there is provided use of an NGF-binding polypeptide comprising at least a portion of the amino acid sequence of Figure 1, or a functional analogue of such a polypeptide, in the preparation of a medicament for the prevention or treatment of a respiratory disorder in a subject. The polypeptide may consist of the amino acid sequence of Figure 1. Alternatively, the polypeptide may comprise the full amino acid sequence of Figure 1.

The term "functional analogue", as used throughout this specification, indicates an NGF-binding polypeptide having an amino acid sequence, for example the amino acid sequence of Figure 1 or Figure 2, in which some or all of the amino acids has been replaced by an amino acid which does not significantly affect the NGF-binding function of the polypeptide, i.e. a 'conservative" substitution. For example, a single amino acid may be substituted in the amino acid sequence of Figure 1 without altering the NGF-binding properties of the polypeptide. This "substituted" amino acid sequence would be a functional analogue of the polypeptide consisting of the amino acid sequence of Figure 1.

Advantageously, the use according to the first aspect of the invention allows the control and relief of asthmatic symptoms by tackling the underlying mechanism of the asthmatic event rather than merely acting as a bronchodilator.

According to a second aspect of the invention, there is provided use of a NGF-binding polypeptide comprising at least a portion of the amino acid sequence of Figure 2, or a functional analogue of such a polypeptide, in the preparation of a medicament for the prevention or treatment of a respiratory disorder in a subject. The polypeptide may consist of the amino acid sequence of Figure 2. Alternatively, the polypeptide may comprise the full amino acid sequence of Figure 2.

According to a third aspect of the invention, there is provided use of a NGF-binding chemical species, which is a topographical analogue of a polypeptide comprising at least a portion of the amino acid sequence of Figure 1 or Figure 2, or of a functional analogue of such a polypeptide, in the preparation of a medicament for the prevention or treatment of a respiratory disorder in a subject. The topographical analogue may be a topographical analogue of a polypeptide consisting of the amino acid sequence of Figure I or Figure 2.

The term "topographical analogue", as used throughout this specification, means a species which has three-dimensional characteristics substantially identical or similar to those of a polypeptide comprising at least a portion of the amino acid sequence of Figure 1 or Figure 2, or of a functional analogue of such a polypeptide (i.e. a species which is a peptidomimetic).

In embodiments according to the first, second or third aspects of the invention, the medicament may contain less than 50 ng of a polypeptide comprising at least a portion of the amino acid sequence of Figure 1 or Figure 2, or of a functional analogue of such a polypeptide, or of a topographical analogue of a polypeptidecomprising at least a portion of the amino acid sequence of Figure 1 or Figure 2, or of a topogaphical analogue of a functional analogue of such a polypeptide. Alternatively, the medicament may contain at least 50 ng, preferably at least 200ng, most preferably at least 800ng of a NGF-binding polypeptide comprising at least a portion of the amino acid sequence of Figure 1 or Figure 2, or of a functional analogue of such a polypeptide, or of a topographical analogue of a polypeptide comprising at least a portion of the amino acid sequence of Figure 1 or Figure 2, or of a topographical analogue of a functional analogue of such a polypeptide.

The respiratory disorder may be an inflammatory disorder, preferably asthma cough or inflammatory rhinitis. The subject may be a mammal and is, preferably, a human subject.

The use according to the first, second or third aspects of the invention may be continued for at least two days, preferably for at least six days.

According to a fourth aspect of the invention, there is provided use of a nucleotide sequence encoding for at least a portion of the amino acid sequence of Figure 1 or Figure 2 in the preparation of a medicament for the treatment of a respiratory disorder in a subject.

The respiratory disorder may be an inflammatory disorder, preferably asthma, cough or inflammatory rhinitis. The subject may be a mammal and preferably is a human subject.

The use according to the fourth aspect of the invention may be continued over at least two days, preferably at least six days.

According to a fifth aspect of the invention, there is provided a pharmaceutical composition for use in the prevention or treatment of a respiratory disorder in a subject, the composition comprising:
a) an effective amount of an NGF-binding polypeptide comprising at least a portion of the amino acid sequence of Figure 1 or Figure 2, or of a functional analogue of such a polypeptide, or pharmaceutically acceptable salts thereof; and
b) a pharmaceutically acceptable adjuvant, carrier or vehicle.

The polypeptide may consist of the amino acid sequence of Figure 1 or Figure 2. The effective amount may be less than 50 ng/(kg subject). Alternatively, the effective amount may be at least 50 ng/(kg subject), preferably at least 200 ng/(kg subject), most preferably at least 800 ng/(kg subject).

According to a sixth aspect of the invention, there is provided a pharmaceutical composition for use in the prevention or treatment of a respiratory disorder in a subject, the composition comprising:
a) an effective amount of a topographical analogue of a NGF-binding polypeptide comprising at least a portion of the amino acid sequence of Figure 1 or Figure 2, or of a topographical analogue of a functional analogue of such a polypeptide, or pharmaceutically acceptable salts of such a topographical analogue; and
b) a pharmaceutically acceptable adjuvant, carrier or vehicle.

The topographical analogue may be a topographical analogue of a polypeptide consisting of the amino acid sequence of Figure 1 or Figure 2. The effective amount may be less than 50 ng/(kg subject). Alternatively, the effective amount may be at least 50 ng/(kg subject), preferably at least 200 ng/(kg subject), most preferably at least 800 ng/(kg subject).

According to a seventh aspect of the invention, there is provided a pharmaceutical composition for use in the prevention or treatment of a respiratory disorder in a subject, the composition comprising:
a) an effective amount of a nucleotide sequence encoding for at least a portion of the amino acid sequence of Figure 1 or Figure 2, or of a nucleotide sequence encoding a functional analogue of the amino acid sequence of Figure 1 or Figure 2, or pharmaceutically acceptable salts thereof; and
b) a pharmaceutically acceptable adjuvant, carrier or vehicle.

In embodiments according to the fifth, sixth and seventh aspects of the invention, the respiratory disorder may be an inflammatory disorder, preferably asthma, cough or inflammatory rhinitis. The subject may be a mammal and preferably is a human subject.

The pharmaceutically acceptable adjuvant, carrier or vehicle of the third or fourth aspects of the invention may be selected from: ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical composition may be administered orally or parenterally.

Where the pharmaceutical composition is administered orally, it may be in the form of a capsule or a tablet which may comprise lactose and/or corn starch. The pharmaceutical composition may further comprise a lubricating agent. Preferably magnesium stearate. The pharmaceutical composition may be in the form of an aqueous suspension or solution which may further comprise an emulsifying and/or suspending agent. The pharmaceutical composition may comprise sweetening, flavouring and/or colouring agents.

The pharmaceutical composition may be administered by injection, by needle-free device, by inhalation spray, by fine powder inhalation, topically, nasally, buccally, or via an implanted reservoir. Preferred methods of delivery are by oral or nasal inhalation spray or by oral or nasal fine powder inhalation.

Where the pharmaceutical composition is administered by injection or needle-free device, it may be in the form of a sterile injectable preparation or a form suitable for administration by needle-free device. The sterile injectable preparation or form suitable for administration by needle-free device may be an aqueous or an oleaginous suspension, or a suspension in a non-toxic parenterally-acceptable diluent or solvent. The aqueous suspension may be prepared in mannitol, water, Ringer's solution or isotonic sodium chloride solution. The oleaginous suspension may be prepared in a synthetic monoglyceride, a synthetic diglyceride, a fatty acid or a natural pharmaceutically-acceptable oil. The fatty acid may be an oleic acid or an olive acid glyceride derivative. The natural pharmaceutically-acceptable oil may be an olive oil, a castor oil, or a polyoxyethylated olive oil or castor oil. The oleaginous suspension may contain a long-chain alcohol diluent or dispersant, preferably Ph. Helv.

Where the pharmaceutical composition is administered topically, it may be an ointment comprising a carrier selected from mineral oil, liquid petroleum, white petroleum, propylene glycol, polyoxyethylene-polyoxypropylene compounds, emulsifying wax and water. Alternatively, it may be a lotion or cream comprising a carrier selected from mineral oil, sorbitan monostearate, polysorbate 60, cetyl esters wax, cetearyl alcohol, 2-octyldodecanol, benzyl alcohol and water.

Where the pharmaceutical composition is administered nasally, it may be administered by nasal aerosol and/or inhalation, including inhalation of a fine powder.

According to an eight aspect of the invention, there is provided a method of preventing or treating a respiratory disorder, the method comprising administering an effective amount of a polypeptide comprising at least a portion of the amino acid of Figure 1 or Figure 2, or of a functional analogue of such a polypeptide, to an individual in need of such prevention or treatment. The polypeptide may consist of the amino acid sequence of Figure 1 or Figure 2. Alternatively, two polypeptides may comprise the full amino acid sequence of Figure 1 or Figure 2.

The effective amount may be less than 50 ng/(kg subject). Alternatively, the effective amount may be at least 50 ng/(kg subject), preferably at least 200 ng/(kg subject), most preferably at least 800 ng/(kg subject).

According to a ninth aspect of the invention, there is provided a method of preventing or treating a respiratory disorder, the method comprising administering an effective amount of a topogaphical analogue of a NGF-binding polypeptide comprising at least a portion of the amino acid sequence of Figure 1 or Figure 2, or of a topographical analogue of a functional analogue of such a polypeptide, to an individual in need of such prevention or treatment. The topographical analogue may be a topographical analogue of a polypeptide consisting of the amino acid sequence of Figure 1 or Figure 2.

The effective amount may be less than 50 ng/(kg subject). Alternatively, the effective amount may be at least 50 ng/(kg subject), preferably at least 200 ng/(kg subject), most preferably at least 800 ng/(kg subject).

According to a tenth aspect of the invention, there is provided a method of preventing or treating a respiratory disorder, the method comprising administering an effective amount of a nucleotide encoding a NGF-binding polypeptide comprising at least a portion of the amino acid sequence of Figure I. or Figure 2, or of a nucleotide encoding a functional analogue of such an amino acid sequence, to an individual in need of such prevention or treatment.

The respiratory disorder may be an inflammatory disorder, preferably asthma, cough or inflammatory rhinitis. The individual may be a mammal and is preferably a human individual. The method may be carried out over at least two days, preferably over at least six days.

### ABBREVIATIONS

The following abbreviations are used throughout this specification:
- AHR =: airway hyperreactivity
- Alum =: aluminium hydroxide
- BALF =: bronchoalveolar lavage fluid
- Cps =: capsaicin
- EtOH =: ethanol
- IgE =: immunoglobulin E
- i.p. =: intraperitoneal administration
- i.n. =: intranasal administration
- inh =: inhalative aerosol challange
- i.t. =: intratracheal administration
- lq.N₂ -: liquid nitrogen
- MCh =: methacholine
- NHR =: neuronal hyperresponsiveness
- OVA =: ovalbumin
- PES =: phosphate buffered saline
- Tb =: time of break
- TMEF =: tidal midexpiratory flow
- vehicle =: saline solution

### BRIEF DESCRIPTION OF THE DRAWINGS

Embodiments of the invention will now be described, by way of example only, with reference to the accompanying Figures 1-20 in which:
Figure 1 shows the amino acid sequence of polypeptide A, to be used in accordance with the invention;
Figure 2 shows the amino acid sequence of polypeptide B, to be used in accordance with the invention;
Figure 3 shows the study design for Example 1 below;
Figure 4 shows the time of break following capsaicin challenge in mice (mean ± SEM);
Figure 5 shows statistical evaluation of capsaicin responsiveness (horizontal bars = median of each group);
Figure 6 shows AHR in mice in response to exposure with aerosolized methacholine (horizontal bars represent median values);
Figure 7 shows ovalbumin-induced airway obstruction in mice (mean values ± SEM);
Figure 8 shows ovalbumin-induced airway obstruction after A: 0 minutes, B: 3-6 minutes, C: 12-15 minutes and D: 18-21 minutes after ovalbumin instillation (horizontal bars represent median values);
Figure 9 shows ovalbumin-induced airway obstruction relative to baseline values (mean values ± SEM);
Figure 10 shows the number of eosinophils in bronchoalvolar lavage fluid 24 hours after allergen challenge in mice (horizontal bars represent median values);
Figure 11 shows the number of lymphocytes in bronchoalveolar lavage fluid 24 hours after allergen challenge in mice (horizontal bars represent median values);
Figure 12 shows the study design for Example 2 below; Figure 13 shows the time of break following capsaicin challenge in mice at day 28 of the study (mean ± SEM);
Figure 14 shows statistical evaluation of capsaicin responsiveness at day 28 for A: 50 µg/ml capsaicin, B: 100 µg/ml capsaicin, C: 500 µg/ml capsaicin and D: 1000 µg/ml capsaicin (horizontal bars =median of each group);
Figure 15 shows the time of break following capsaicin challenge in mice at day 36 of the study (mean ± SEM);
Figure 16 shows statistical evaluation of capsaicin responsiveness at day 36 for A: 10 µg/ml capsaicin, B: 50 µg/ml capsaicin, C: 100 µg/ml capsaicin and D: 500 µg/ml capsaicin (horizontal bars = median of each group);
Figure 17 is a comparison between the capsaicin-induced sensory irritation measured on day 28 and day 36 (horizontal bars = median of each group);
Figure 18 shows AHR in mice in response to exposure with aerosolized methacholine (horizontal bars represent median values);
Figure 19 shows the cumulative measurements of AHR in mice in response to exposure with aerosolized methacholine, from Example 1 and Example 2 (horizontal bars represent median values); and
Figure 20 shows the number of eosinophils in bronchoalveolar lavage fluid 24 hours after allergen challenge in mice (horizontal bars represent median values).

### MODES OF CARRYING OUT THE INVENTION

### 1. Effect of intratracheal application of polypeptides A or B

The aim of the study was to investigate the influence of polypeptides A and B after local intratracheal application in a mouse model of allergic asthma. Therefore, the allergen-induced early phase reaction (EAR) in response to Ovalbumin (OVA), the airway hyperreactivity (AHR) to methacholine and the neuronal hyperresponsiveness (NHR) to capsaicin were measured by head-out bodyplethysmography. Allergic inflammation was assessed by total and differential cell count of bronchoalveolar lavage (BAL) cells, determination of cytokine levels in the BAL fluid (IL-4, IL-5 and IFN-y), and total serum IgE levels.

The study was performed according to the previously described OVA-asthma model (Päth et al. 2002, Am. J. Respir. Crit. Care Med.).

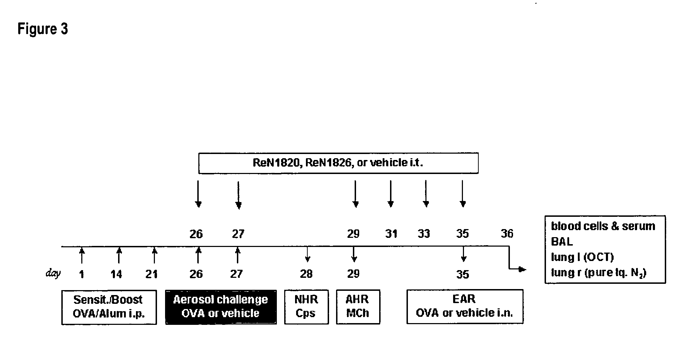

All animals (female Balb/c mice, age 6-8 weeks) were sensitized systemically with OVA plus adjuvant (aluminium hydroxide) and were boosted and challenged as follows. The positive and the negative control groups were treated intratracheally with vehicle (50 µl saline) before inhalative OVA challenge and then every second day from day 29 to day 35,2 hours before last allergen challenge. Each group was treated correspondingly with polypeptide A or polypeptide B (200 ng of each substance/treatment/animal diluted in 50 µl saline) (Table 1, Figure 3).

**Table 1: Sensitization, challenge and treatment compounds for the study of Example 1.**

| **Group number** | **Sensitization/Boost** | **Challenge/ EAR** | **Treatment** | **Total** |
|---|---|---|---|---|
| Negative n= 15 Control | OVA/Alum i.p. | vehicle inh. /i.n. | | vehicle i.t. |
| Positive n= 15 Control | OVA/Alum i.p. | OVA inh. / i.n. | | vehicle i.t. |
| Polypeptide n= 15 B | OVA/Alum i.p. | OVA inh. / i.n. | | Polypeptide B i.t. (200ng/mouse/treatment) |
| Polypeptide n= 15 A | OVA/Alum i.p. | OVA inh. / i.n. | | Polypeptide A i.t (200ng/mouse/treatment) |

For antigen challenge, the positive control group and all the treatment groups were exposed to a defined OVA aerosol. The negative control group were exposed to the vehicle accordingly.

### Neuronal hyperresponsiveness

The neuronal hyperresponsivencss (NHR) is an indicator of sensory hyperresponsiveness in the airways and can be indirectly measured after capsaicin challenge (staged protocol). Capsaicin is a specific agonist for the vanilloid receptor 1 (VR1) expressed in peripheral sensory nerves. Stimulation of the VRI results in a centrally mediated change in the breathing pattern of the animals. The prolonged time of braking (Tb) before expiration is therefore a selective marker for sensory irritation of the airways (Alarie Y. (1998) Arch Toxicol. 72 277-282).

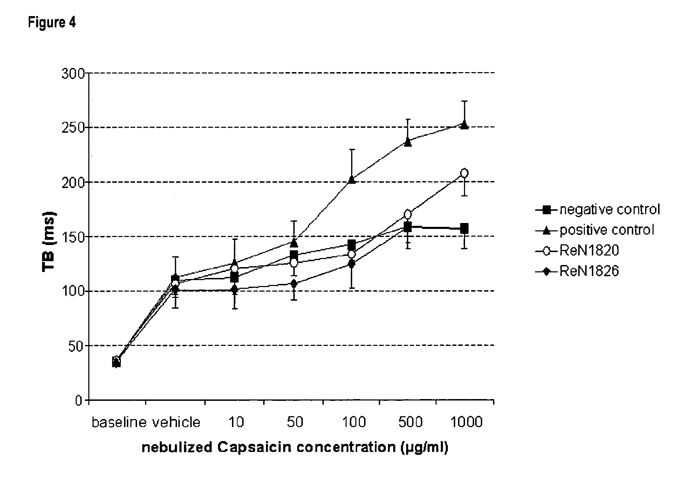

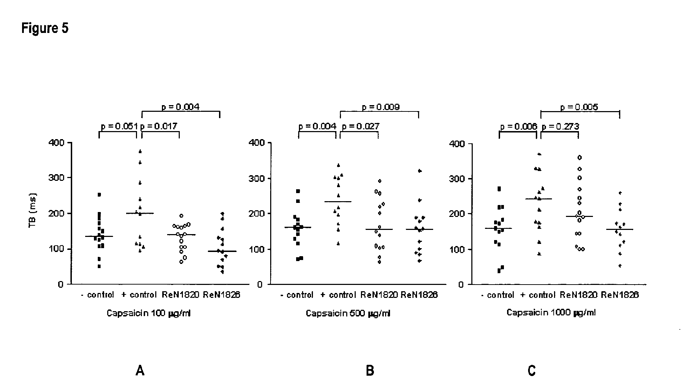

Tb is increased after nebulization of the diluent (10% EtOH/PBS) itself as has been described previously (Kerzel et al (2003) Am. J. Cell. Mol. Biol. 28 170-178). Airway reaction to EtOH/PBS did not vary between the examined groups. Following capsaicin challenge, all mice showed a dose-dependent rise in Tb (Figure 4). At concentrations higher than 500 µg/ml, OVA-sensitised and -challenged mice (positive control group) reacted significantly more strongly than sensitised and not challenged mice (negative control), indicating hyperreactivity to capsaicin. Local treatment with polypeptide A reduced the response of OVA-sensitised and -challenged mice to capsaicin, whereas the intratracheal administration of polypeptide A almost prevented sensory, irritation following capsaicin challenge (Figure 5).

### Airway hyperreactivity

The term "airway hyperreactivity" (AHR) describes an abnormal increased reaction of the airways to many unspecific stimuli like smoke, cold air and ozone. AHR is a characteristic phenomenon of patients with allergic asthma. To assess AHR in mice, airflow limitation expressed as reduced tidal midexspiratory flow (TMEF) was measured by head-out bodyplethysmography after inhalation of aerosolized methacholine (MCh) (staged protocol).

The effective dose needed to decrease the TMEF to 50% of basa values (ED₅₀) was lower in the group of Ovalbumin (OVA)-immunized and challenged animals (positive control) compared to the animals of the negative control group representing AHR.

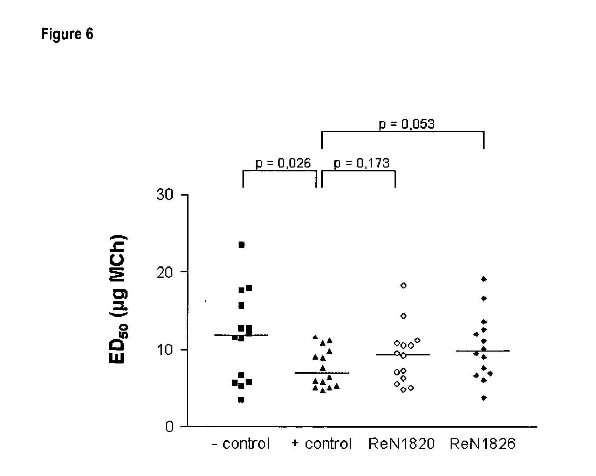

After treatment with either polypeptide A or polypeptide B, a tendency to a decrease in AHR was seen (expressed as an increase in ED₅₀ compared to the positive control group). This change was not statistically significant (Figure 6).

### Early phase reaction to allergen challenge

Allergen contact itself results in airflow limitation (reduced TMEF) referred to as early phase response (EAR). The EAR can be measured after intranasal application of OVA in OVA-sensitized animals by head-out bodyplethysmography.

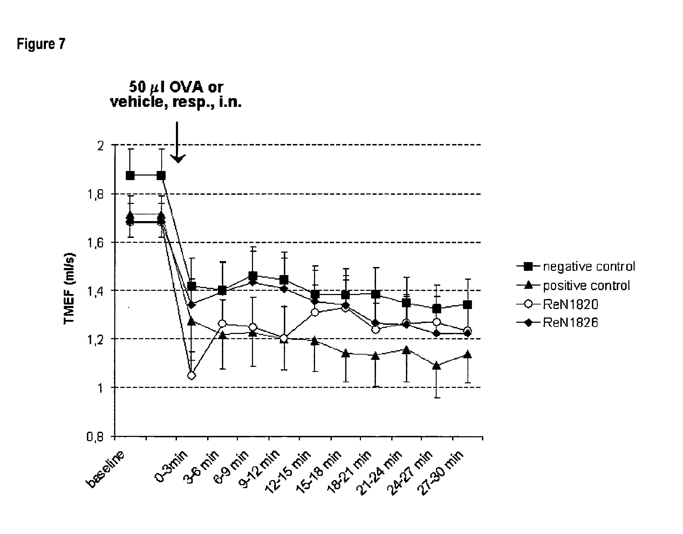

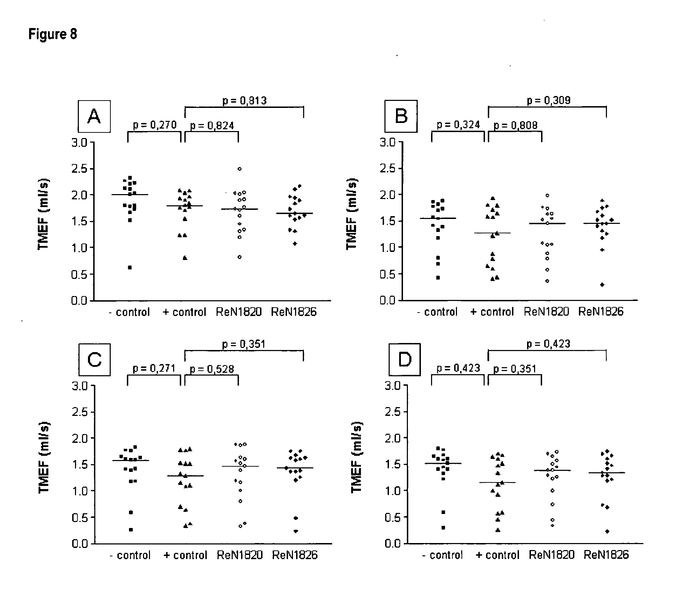

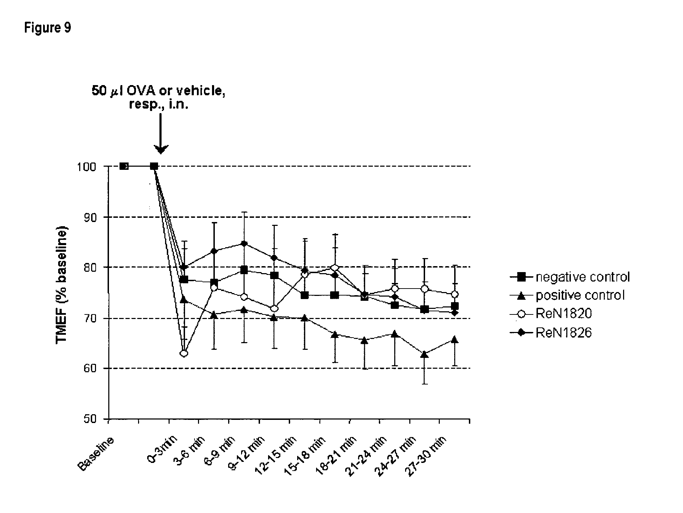

Instillation of allergen in the airways of OVA-sensitized and -challenged animals(positive controls) caused a decrease in absolute (Figures 7 and 8) and relative TMEF values (Figure 9) within a few minutes, as expected. After pretreatment with polypeptide A or polypeptide B, tendencies toward a reduction of OVA-induced airflow limitation were observed. These alterations were not statistically significant.

### Airway inflammation

The allergic inflammatory response was examined 24 hours after the last OVA provocation. To evaluate airway inflammation, bronchoalvelolar lavages (BAL) were performed. The recovered volume of BAL fluid (BALF) was defined and the number of cells in the BALF was automatically assessed using an Act8 cell counter. After centrifugation, BALF supernatant was removed and stored at -20°C for further cytokine analysis. BALF cells were resuspended in RPMI medium and cytospins were performed for differential cell count. After May-Grünwald-Giemsa staining, a total number of 600 cells/slide were analysed, according to their morphological criteria, by light microscopy.

The total cell number was significantly enhanced in the positive control group as expected. Cell numbers did not differ between the positive controls and the animals treated with the polypeptides A or B (data not shown).

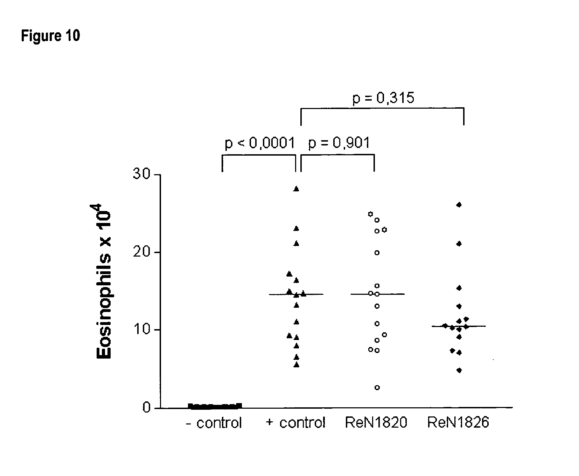

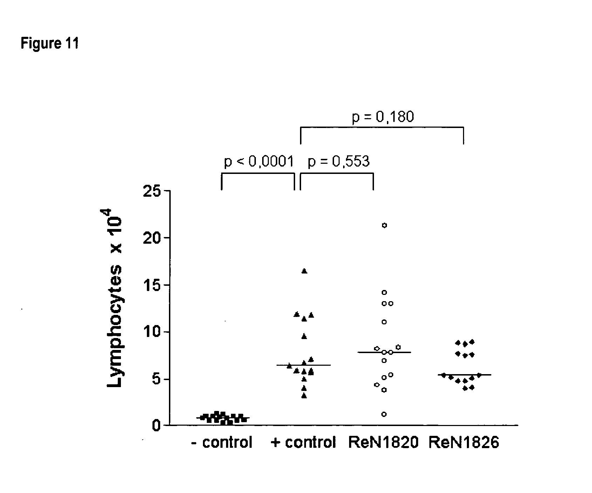

Eosinophils and lymphocytes are known to play a major role in the pathogenesis of allergic inflammation. Differential cell count of BALF cells revealed a significant increase of eosinophils and lymphocytes in the BAL of the positive control animals. Treatment with polypeptide B did not influence the allergen-dependent influx of eosinophils and had no effect on lymphocyte infiltration of the airways, whereas the number of both cell types was slightly decreased in animals treated with polypeptide A compared to the positive control group (not statistically significant) (Figures 10 and 11).

The total number of macrophages and neutrophils were comparable in all examined groups. As expected, allergen challenge had no influence on the number of macrophages nor on the numbers of neutrophils (Data not shown).

### 2. Effect of intranasal application of polypeptide A

The aim of the study was to investigate the influence of polypeptide A after local intranasal application in a mouse model of allergic asthma. Therefore, airway hyperreactivity to methacholine and the neuronal hyperresponsiveness to capsaicin were measured by head-out bodyplethysmography. Allergic inflammation was assessed by total and differential cell count of bronchoalveolar lavage (BAL) cells. The study was performed according to the previously described OVA-asthma model, as above.

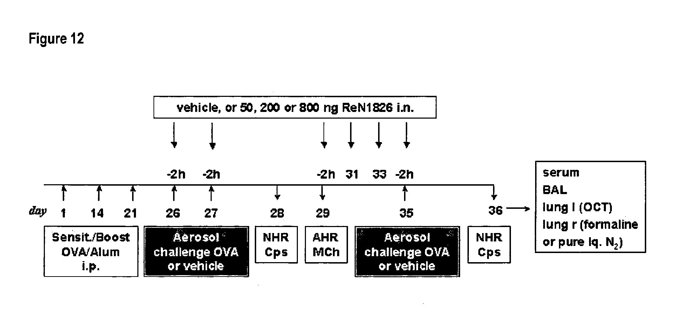

All animals (female Balb/c mice, age 6-8 weeks) were sensitised systemically with ovalbumin plus adjuvant (aluminium hydroxide) and were boosted and challenged as follows. The positive and the negative control groups were treated intranasally with vehicle (50 µl saline) before inhalative ovalbumin challenge and every second day from day 29 to day 35, 2 hours before last allergen challenge. The remaining groups have been treated correspondingly with polypeptide A (50, 200, or 800 ng /treatment/animal diluted in 50 µl saline) (Table 2, Figure 12).

**Table 2: Sensitization, challenge and treatment compounds for the study of Example 2.**

| **Group** | **Sensitization/Boost** | **Challenge number** | **Treatment** | **Total** |
|---|---|---|---|---|
| Negative n=16 Control | OVA/Alum i.p. | vehicle inh. | | vehicle i.n. |
| Positive n=16 Control | OVA/Alum i.p. | OVA inh. | | vehicle i.n. |
| 50 ng =16 polypeptide A | OVA/Alum i.p. | OVA inh. | | 50 ng polypeptide A n |
| | | | i.n.(x ng/ mouse/treatment) | |
| 200 ng n = 16 polypeptide A | OVA/Alum i.p. | OVA inh. | | 200 ng polypeptide |
| | | | A i.n.(x ng/ mouse/treatment) | |
| 800 ng n=16 polypeptide A | OVA/Alum i.p. | OVA inh. | | 800 ng polypeptide |
| | | | A i.n.(x ng/ mouse/treatment) | |

For antigen challenge, both the positive control group and all treatment groups have been exposed to a defined OVA aerosol. The negative control group has been exposed to the vehicle accordingly.

### Neuronal hyperresponsiveness

The neuronal hyperresponsiveness (NHR) was measured on day 28 and day 36 (Figure 12). As described above, NHR is an indicator of sensory hypenesponsiveness in the airways and can be indirectly measured after capsaicin challenge (staged protocol). The prolonged time of braking (Tb) before expiration is a selective marker for sensory irritation of the airways (Alarie Y, (1998) Arch Toxicol. 72 277-282).

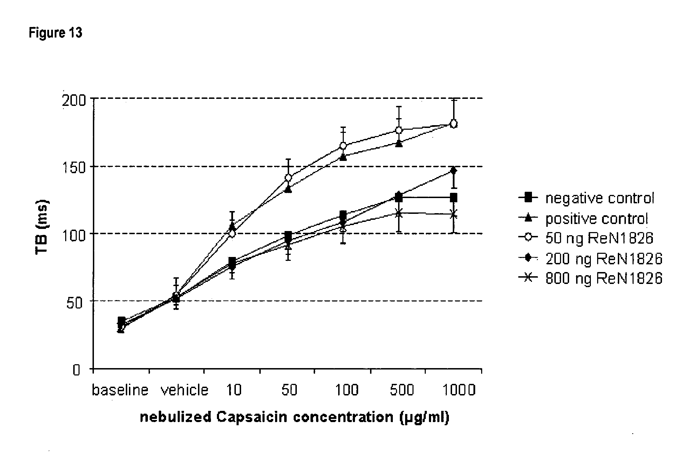

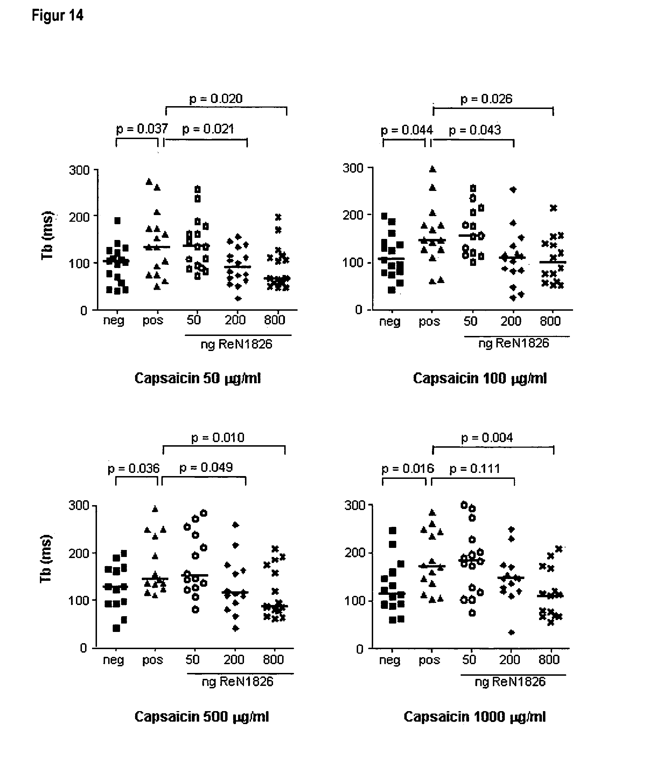

Airway reaction to EtOH/PBS did not vary between the examined groups. Following capsaicin challenge, all mice showed a dose-dependent rise in Tb (Figure 13 - Figure 16). Measurements on day 28 demonstrate that at concentrations higher than 10 µg/ml, OVA-sensitized and -challenged mice (positive control group) reacted significantly more strongly than sensitized and not challenged mice (negative control) indicating hyperreactivity to capsaicin. Treatment of the animals with polypeptide A on two consecutive days caused a dose-dependent decrease of Tb compared to the positive control (Figure 13 and Figure 14). Although 200 ng and 800 ng polypeptide A almost prevented the development of NHR, administration of 50 ng polypeptide A had no effect on sensory irritation following capsaicin challenge. The difference between the 50 ng treatment group and the 200 ng treatment group was statisticallysignificant (p < 0.05), but there was no statistical significance between the 200 ng and 800 ng treatment group (Figure 14).

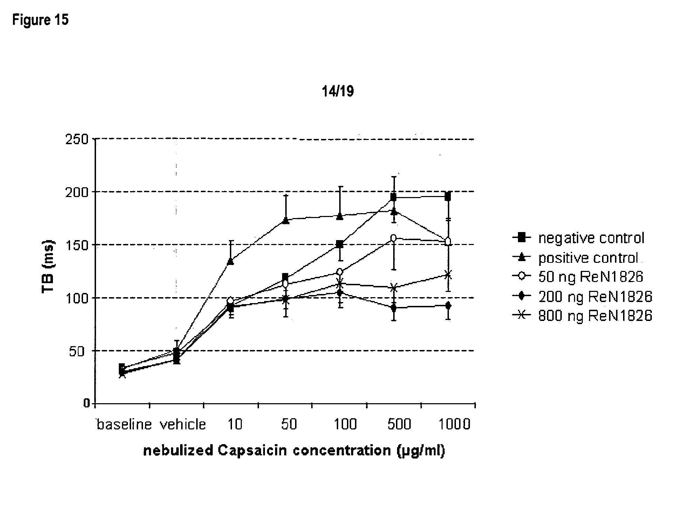

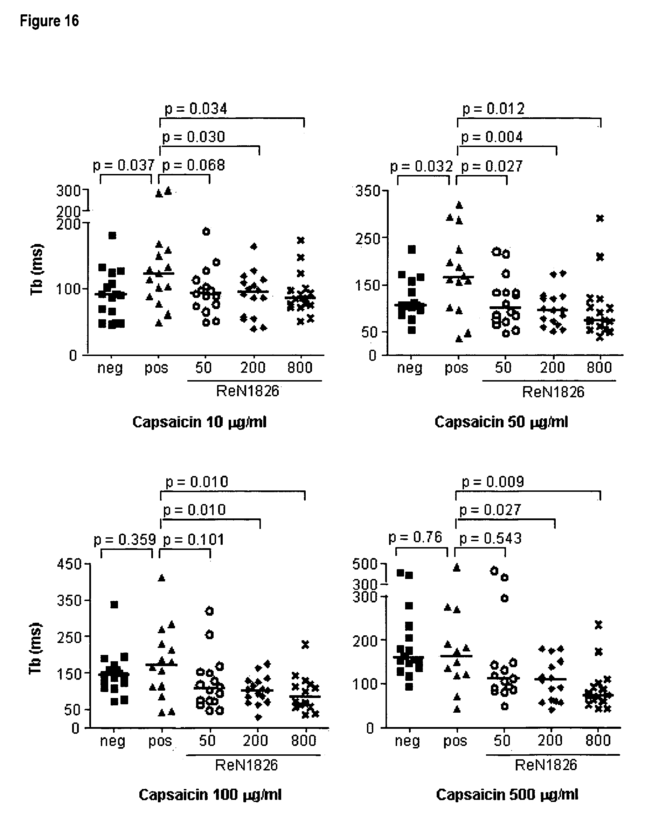

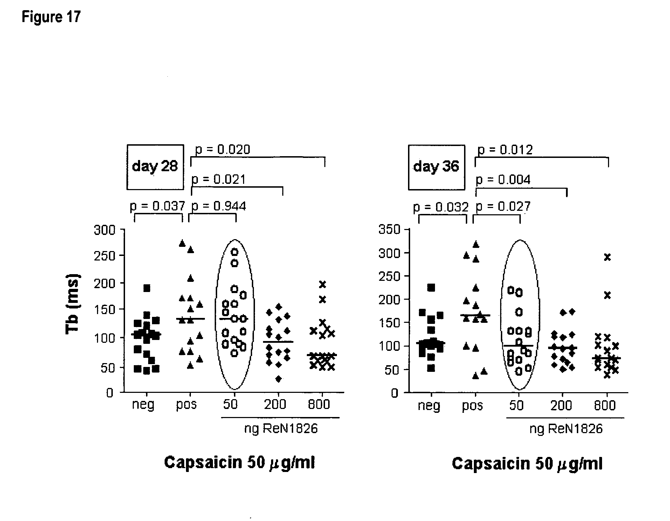

In addition to the measurements on day 28, a lung function test in response to capsaicin was performed on day 36 after prolonged intranasal application of the test substance (Figure 15 - 17). Compared to day 28, the reaction of the negative controls to increasing doses of capsaicin was enhanced which might be explained by the consecutive intranasal treatment. However, at lower capsaicin doses, significant differences in the response between the negative arid positive controls could be observed. Treatment with 200 ng and 800 ng polypeptide A could prevent the sensory irritation to capsaicin comparable to the previously described results on day 28 (Figure 16). In contrast to day 28, administration of six doses of 50 ng polypeptide A also reduced the capsaicin-induced increase in Tb when animals were exposed to the lower doses of capsaicin (day 36) (p < 0.05 at 50 µg/ml capsaicin) (Figure 17, circled values), indicating that a prolonged treatment with polypeptide A can reduce a preexisting neuronal hyperresponsiveness.

### Airway hyperreactivity

As described above, the term "airway hyperreactivity" (AHR) describes an abnormal increased reaction of the airways to many unspecific stimuli like smoke, cold air and ozone. To assess AHR in mice, airflow limitation expressed as reduced tidal midexpiratory flow (TMEF) was measured by head-out bodyplethysmography after inhalation of aerosolized methacholine (MCh) (staged protocol).

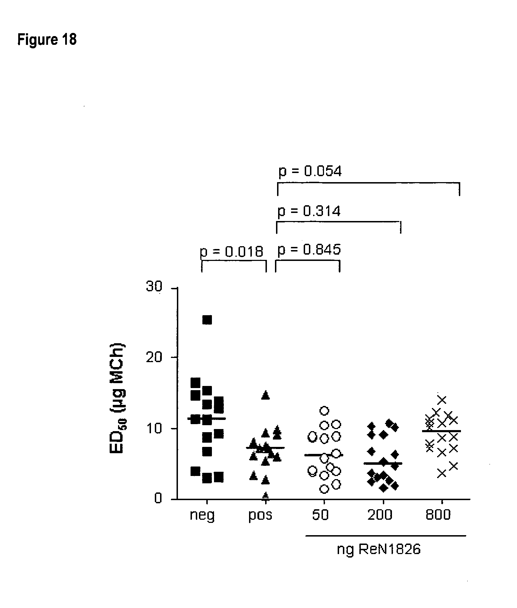

The effective dose needed to decrease the TMEF to 50% of basal values (ED₅₀) was lower in the group of ovalbumin-imnunized and challenged animals (positive control) compared to the animals of the negative control group representing AHR. After treatment with polypeptide A, a strong tendency to a decrease in AHR was seen (day29; expressed as an increase in ED₅₀ compared to the positive control group). This change was not statistically significant (p = 0.054) (Figure 18).

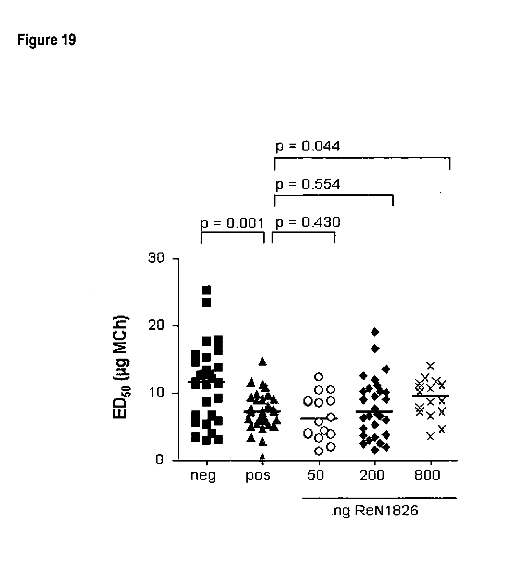

The cumulative results of the study into the effect of intratracheal application of polypeptide A (Example 1 above) and this study into the effect of intranasal application of polypeptide A demonstrate that the effect of polypeptide A to prevent AHR is dose-dependent (Figure 19) and statistically significant (p < 0.05) for the group treated with 800 ng polypeptide A.

### Airway inflammation

The allergic inflammatory response was examined 24 hours after the last ovalbumin provocation. To evaluate airway inflammation, bronchoalvelolar lavages (BAL) were performed. The recovered volume of BAL fluid (BALF) was defined and the number of cells in the BALF was automatically asserssed using a Act8 cell counter, as above. After centrifugation, BALF supernatant was removed and stored at -20°C for further cytokine analysis. BALF cells were resuspended in phosphate-buffered saline (PBS) and cytospins were performed for differential cell count. After May-Grünwald-Giemsa staining, a total number of 600 cells/slide were analysed according to their morphological criteria by light microscopy.

The total cell number was significantly enhanced in the positive control group as expected. Cell numbers did not differ between the positive controls and the animals treated with polypeptide A (data not shown).

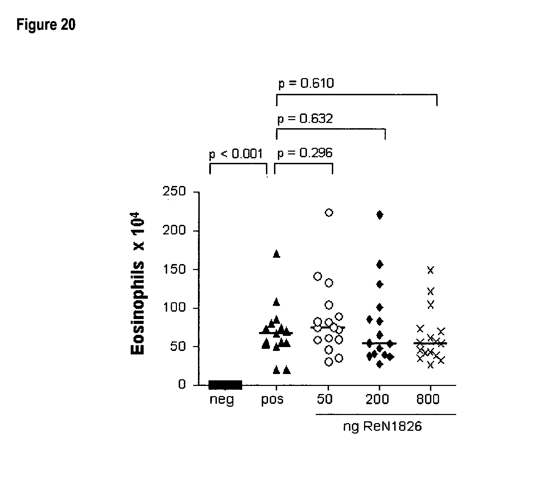

Lymphocytes and eosinophils are known to play a major role in the pathogenesis of allergic inflammation. Differential cell count of BALF cells revealed a significant increase of lymphocytes and eosinophils in the BAL of the positive control animals. The number of eosinophils was slightly decreased in animals treated with polypeptide A compared to the positive control group (not statistically significant) (Figure 20).

The total number of macrophages and neutrophils were comparable in all examined groups (with exception to the number of neutrophils in the 50 ng study group). Asexpected, allergen challenge had no influence on the number of macrophages nor on the numbers of neutrophils (data not shown).

### Summary

The applicants have shown that both polypeptide A and polypeptide B significantly reduce neuronal hyperresponsiveness to capsaicin and show non-significant tendencies to decrease airway hyperreactivity to methacholine and to impair the early phase response to ovalbumin challenge. In the case of polypeptide A, the reduction in neuronal hyperresponsiveness to capsaicin is concentration dependent, effective after two doses where polypeptide A is administered in a high dose and effective after six doses where polypeptide A is administered in a lower dose. Polypeptide A also shows a significant tendency to decrease airway hyperreactivity to methacholine where a high dose of polypeptide A is administered. Finally, polypeptide A shows a non-significant tendency to reduction eosinophils in BALF.

## Claims

1. Use of a NGF binding polypeptide comprising or consisting of at least a portion of the amino acid sequence of Figure 1, or a functional analogue of such a polypeptide, or of a nucleic acid molecule encoding said polypeptide or said functional analogue, in the preparation of a medicament for the prevention or treatment of a respiratory disorder in a subject.

2. Use according to claim 1 wherein the polypeptide consists of the amino acid sequence of Figure 1.

3. Use of a NGF binding polypeptide comprising or consisting of at least a portion of the amino acid sequence of Figure 2, or a functional analogue of such a polypeptide or of a nucleic acid molecule encoding said polypeptide or said functional analogue, in the preparation of a medicament for the prevention or treatment of a respiratory disorder in a subject.

4. Use according to claim 3 wherein the polypeptide consists of the amino acid sequence of Figure 2.

5. Use of a NGF binding chemical species, which is a topographical analogue of a polypeptide comprising or consisting of at least a portion of the amino acid sequence of Figure 1 or Figure 2, or of a functional analogue of such a polypeptide, in the preparation of a medicament for the prevention or treatment of a respiratory disorder in a subject.

6. Use according to claim 5 wherein the topographical analogue is a topographical analogue of a polypeptide consisting of the amino acid sequence of Figure 1 or Figure 2.

7. Use according to any preceding claim wherein the medicament contains less than 50 ng of a NGF binding polypeptide comprising or consisting of at least a portion of the amino acid sequence of Figure 1 or Figure 2, or of a functional analogue of such a polypeptide, or of a topographical analogue of a polypeptide comprising or consisting of at least a portion of the amino acid sequence of Figure 1 or Figure 2, or of the topographical analogue of a functional analogue of such a polypeptide.

8. Use according to any of claims 1 to 6 wherein the medicament contains at least 50 ng of a NGF binding polypeptide comprising or consisting of at least a portion of the amino acid sequence of Figure 1 or Figure 2, or of a functional analogue of such a polypeptide, or of a topographical analogue of a polypeptide comprising or consisting of at least a portion of the amino acid sequence of Figure 1 or Figure 2, or of a topographical analogue of a functional analogue of such a polypeptide.

9. Use according to claim 8 wherein the medicament contains at least 200 ng of a NGF binding polypeptide comprising or consisting of at least a portion of the amino acid sequence of Figure 1 or Figure 2, or of a functional analogue of such a polypeptide, or of a topographical analogue of a polypeptide comprising or consisting of at least a portion of the amino acid sequence of Figure 1 or Figure 2, or of a topographical analogue of a functional analogue of such a polypeptide.

10. Use according to claim 8 or 9 wherein the medicament contains at least 800 ng of a NGF binding polypeptide comprising or consisting of at least a portion of the amino acid sequence of Figure 1 or Figure 2, or of a functional analogue of such a polypeptide, or of a topographical analogue of a polypeptide comprising or consisting of at least a portion of the amino acid sequence of Figure 1 or Figure 2, or of a topographical analogue of a functional analogue of such a polypeptide.

11. Use according to any of claims 1 to 10 wherein the use is continued over at least two days.

12. Use according to claim 11 wherein the use is continued over at least six days.

13. A pharmaceutical composition preferably for use in the prevention or treatment of a respiratory disorder in a subject, the composition comprising:
(a) an effective amount of a NGF binding polypeptide comprising or consisting of at least a portion of the amino acid sequence of Figure 1 or Figure 2, or of a functional analogue of such a polypeptide, or pharmaceutically acceptable salts thereof; and
(b) a pharmaceutically acceptable adjuvant, carrier, excipient or vehicle.

14. A pharmaceutical composition preferably for use in the prevention or treatment of a respiratory disorder in a subject, the composition comprising:
(a) an effective amount of a NGF binding topographical analogue of a polypeptide comprising or consisting of at least a portion of the amino acid sequence of Figure 1 or Figure 2, or of a topographical analogue of a functional analogue of such a polypeptide, or pharmaceutically acceptable salts of such a topographical analogue; and
(b) a pharmaceutically acceptable adjuvant, carrier, excipient or vehicle.

15. A pharmaceutical composition preferably for use in the prevention or treatment of a respiratory disorder in a subject, the composition comprising:
(a) an effective amount of a nucleotide sequence encoding a NGF binding polypeptide comprising or consisting of at least a portion of the amino acid sequence of Figure 1 or Figure 2, or of a nucleotide sequence encoding a functional analogue of said polypeptide, or pharmaceutically acceptable salts thereof; and
(b) a pharmaceutically acceptable adjuvant, carrier, excipient or vehicle.

16. The pharmaceutical composition according to any one of claims 13 to 16 wherein the effective amount is less than 50 ng/(kg subject).

17. The pharmaceutical composition according to any one of claims 13 to 16 wherein the effective amount is at least 50 ng/(kg subject).

18. The pharmaceutical composition according to claim 17 wherein the effective amount is at least 200 ng/(kg subject).

19. The pharmaceutical composition according to claim 17 or 18 wherein the effective amount is at least 800 ng/(kg subject).

20. The pharmaceutical composition according to any of claims 13 and 15 to 19 wherein the polypeptide consists of the amino acid sequence of Figure 1 or Figure 2.

21. The pharmaceutical composition according to any one of claims 14 and 16 to 20 wherein the topographical analogue is a topographical analogue of a polypeptide consisting of the amino acid sequence of Figure 1 or Figure 2.

22. The use according to any one of claims 1 to 12 or the pharmaceutical composition according to any of claims 13 to 21 wherein the respiratory disorder is an inflammatory disorder, and preferably asthma, cough or inflammatory rhinitis.

23. The use of any one of claims 1 to 12 and 22 or the pharmaceutical composition according to any of claims 13 to 22 wherein the subject is a mammal.

24. The use or the pharmaceutical composition according to claim 23 wherein the subject is a human subject.

25. The use of any one of claims 1 to 12 and 22 to 24 or the pharmaceutical composition according to any of claims 13 to 24 wherein the pharmaceutical composition is administered orally.

26. The use or pharmaceutical composition according to claim 25 wherein the pharmaceutical composition is in the form of a tablet or capsule.

27. The use of any one of claims 1 to 12 and 22 to 24 or the pharmaceutical composition according to any of claims 13 to 24 wherein the pharmaceutical composition is administered parenterally.

28. The use or pharmaceutical composition according to claim 27 wherein the pharmaceutical composition is administered by inhalation spray, by fine powder inhalation, by injection or by needle free delivery.

29. The use or pharmaceutical composition according to claim 27 or 28 wherein the pharmaceutical composition is administered by nasal inhalation spray or nasal fine powder inhalation.


### Annex to the application documents - subsequently filed sequences listing

SEQUENCE LISTING

<110> Fraunhofer-Gesellschaft zur Förderung der angewandten
Forschung e.V.

<120> Treatment of respiratory disorders

<130> M 1925 EP

<140> 06 01 0106.0
<141> 2006-05-16

<160> 2

<170> PatentIn version 3.3

<210> 1
<211> 129
<212> PRT
<213> unknown

<220>

<400> 1

Pro Ala Ser Val Gln Leu His Thr Ala Val Glu Met His His Trp Cys
1               5                   10                  15

Ile Pro Phe Ser Val Asp Gly Gln Pro Ala Pro Ser Leu Arg Trp Leu
            20                  25                  30

Phe Asn Gly Ser Val Leu Asn Glu Thr Ser Phe Ile Phe Thr Glu Phe
        35                  40                  45

Leu Glu Pro Ala Ala Asn Glu Thr Val Arg His Gly Cys Leu Arg Leu
    50                  55                  60

Asn Gln Pro Thr His Val Asn Asn Gly Asn Tyr Thr Leu Leu Ala Ala
65                  70                  75                  80

Asn Pro Phe Gly Gln Ala Ser Ala Ser Ile Met Ala Ala Phe Met Asp
                85                  90                  95

Asn Pro Phe Glu Phe Asn Pro Glu Asp Pro Ile Pro Val Ser Phe Ser
            100                 105                 110

Pro Val Asp Thr Asn Ser Thr Ser Gly Asp Pro Val Glu Lys Lys Asp
        115                 120                 125

Glu

<210> 2
<211> 123
<212> PRT

<213>  unknown

<220>

<400>  2

Pro Ala Ser Val Gln Leu His Thr Ala Val Glu Met His His Trp Cys
1               5                   10                  15

Ile Pro Phe Ser Val Asp Gly Gln Pro Ala Pro Ser Leu Arg Trp Leu
            20                  25                  30

Phe Asn Gly Ser Val Leu Asn Glu Thr Ser Phe Ile Phe Thr Glu Phe
        35                  40                  45

Leu Glu Pro Ala Ala Asn Glu Thr Val Arg His Gly Cys Leu Arg Leu
    50                  55                  60

Asn Gln Pro Thr His Val Asn Asn Gly Asn Tyr Thr Leu Leu Ala Ala
65                  70                  75                  80

Asn Pro Phe Gly Gln Ala Ser Ala Ser Ile Met Ala Ala Phe Met Asp
                85                  90                  95

Asn Pro Phe Glu Phe Asn Pro Glu Asp Pro Ile Pro Asp Thr Asn Ser
            100                 105                 110

Thr Ser Gly Asp Pro Val Glu Lys Lys Asp Glu
        115                 120